Europäisches Patentamt

European Patent Office

Office européen des brevets

(19)

(11) **EP 1 476 597 B1**

(12) **EUROPÄISCHE PATENTSCHRIFT**

| | |
|---|---|
| (45) Veröffentlichungstag und Bekanntmachung des Hinweises auf die Patenterteilung:<br>**14.12.2005 Patentblatt 2005/50** | (51) Int Cl.$^7$: **D04H 1/56**, A61F 13/38 |
| (21) Anmeldenummer: 03763852.5 | (86) Internationale Anmeldenummer:<br>**PCT/EP2003/007627** |
| (22) Anmeldetag: **15.07.2003** | (87) Internationale Veröffentlichungsnummer:<br>**WO 2004/007825 (22.01.2004 Gazette 2004/04)** |

(54) **WATTESTÄBCHEN FÜR KOSMETISCHE ODER MEDIZINISCHE ZWECKE ODER FÜR DIE KÖRPERPFLEGE**

COTTON SWAB USED FOR COSMETIC OR MEDICAL PURPOSES OR FOR BODY CARE

COTON-TIGE POUR APPLICATIONS COSMETIQUES OU MEDICALES OU POUR L'HYGIENE CORPORELLE

(84) Benannte Vertragsstaaten:
**AT BE BG CH CY CZ DE DK EE ES FI FR GB GR HU IE IT LI LU MC NL PT RO SE SI SK TR**

(30) Priorität: **15.07.2002 EP 02400033**
**05.05.2003 DE 10321906**

(43) Veröffentlichungstag der Anmeldung:
**17.11.2004 Patentblatt 2004/47**

(73) Patentinhaber: **Paul Hartmann Aktiengesellschaft**
**89522 Heidenheim (DE)**

(72) Erfinder:
• **MANGOLD, Rainer**
**89542 Herbrechtingen (DE)**
• **RÖMPP, Angela**
**73119 Zell u.A. (DE)**

• **MICHELMANN, Jana**
**89522 Heidenheim (DE)**

(74) Vertreter: **Friz, Oliver et al**
**Patentanwälte,**
**Dreiss, Fuhlendorf, Steimle & Becker,**
**Postfach 10 37 62**
**70032 Stuttgart (DE)**

(56) Entgegenhaltungen:
**EP-A- 0 245 017        EP-A- 0 363 533**
**EP-A- 0 402 140        EP-A- 0 405 043**
**EP-A- 0 963 745        WO-A-97/07762**
**US-A- 4 100 324        US-A- 4 755 178**

• **BEECH S R ET AL: "TEXTILE TERMS AND DEFINITIONS, PASSAGE" , TEXTILE TERMS AND DEFINITIONS, MANCHESTER, TEXTILE INSTITUTE, GB, PAGE(S) 239 XP002227582 Seite 239, Absatz 7**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Wattestäbchen für kosmetische oder medizinische Zwecke oder für die Körperpflege, mit einem Stiel, welcher einen Wattekopf bildendes Fasermaterial an zumindest einem seiner Enden aufweist. Solche kosmetische oder medizinische Wattestäbchen dienen zum Reinigen, zum Abschminken oder zum Auftragen kosmetischer Produkte, oder im medizinischen Bereich zum Reinigen von Wunden oder zum Auftragen von Salben.

Wattestäbchen dieser Art sind vielfach bekannt geworden. EP 0 402 140 B1 offenbart ein Wattestäbchen mit einem Wattekopf aus lipophilen Polyolefinmikrofasern. Der Begriff Mikrofasern bleibt aber weitgehend undefiniert.

EP 0 363 533 A1 beschreibt ein Wattestäbchen, dessen Fasermaterial Baumwollfasern und wärmeschmelzbare Bikomponentenfasern aufweist, deren Anteil beispielhaft mit wenigstens 10 Gew.-% angegeben ist. Weitere Hinweise lassen sich dieser Druckschrift nicht entnehmen.

DE 101 23 971 C2 zeigt einen Reinigungsstab zum Reinigen von elektrischen oder elektromechanischen Bauteilen. Er umfasst ein spitz zulaufendes Ende, welches von einem Stück Stoff umgeben ist, und zwar der konischen Form des Stabes folgend. Es entsteht ein versiegeltes konisches Ende an dem Reinigungsstab. Der Stoff kann ein Polyestergewirk oder alternativ ein Polyester-Nylon-Mikrofasermaterial sein.

US 4,100,324 beschreibt eine Fasermischung aus Holzzellstofffasern (Wood Pulp Fibers) und thermoplastischen Mikrofasern eines mittleren Faserdurchmessers von weniger als 10 µm. Dadurch dass diese Fasermischung quasi gleichzeitig mit dem Spinnen der Mikrofasern hergestellt wird, indem nämlich in den Bereich des Spinnkopfs für die Mikrofasern ein Luftstrom sowie ein Zellstoff-Faserstrom eingeströmt wird, entsteht eine Verbindung der Fasern und die Mikrofasern führen zu einer Fixierung der Zellstofffasern, und zwar in einem Zustand, in dem die Mikrofasern sich noch bei erhöhten Temperaturen in einem noch nicht verfestigten Zustand befinden. Die Mikrofasern wirken daher für die Holzzellstofffasern matrixbildend, wobei sie die Holzzellstofffasern gelenkig, also nicht starr einbinden, und zwar auch bei sehr geringen Gehalten an Mikrofasern bis herunter zu 1 Gew.-%. Es können zusätzlich weitere Fasern oder partikelförmiges Material, einschließlich synthetische Fasern wie Nylonfasern und natürliche Fasern wie Baumwolle, Flachs, Jute und Seide, eingebracht werden.

Durch die Herstellung der Fasermischung quasi in situ mit der Bildung der Mikrofasern im Schmelzblas-Prozess (melt blown-Verfahren) verlieren die Mikrofasern ihre Eigenständigkeit in dem Faserprodukt und werden aufgrund des Kontakts mit den Holzzellstoff-Fasern im noch erweichten Zustand verändert und fixiert. Sie lassen sich also nicht mehr als individueller Faserbestandteil oder als Faserart zur Herstellung eines Produkts, d.h. zur Einführung in einen Herstellungsprozess verwenden.

Nach der Lehre dieser Druckschrift US 4,100,324 wird der kombinierte Strom von Schmelzblasmikrofasern und Holzzellstofffasern in den Spalt zweier Vakuumwalzen eingeleitet und dort zu einem Vlies sehr hohen Flächengewichts von 50 g/m$^2$ bis nahezu 200 g/m$^2$ gebildet. Das Fasergemisch ist nach der Vliesbildung in seiner endgültigen Vliesform. Eine anschließende Zerfaserung, also Vereinzelung der Fasern ist nicht mehr möglich.

Des weiteren sind Wattestäbchen bekannt, deren Wattekopf zu 100% aus Baumwollfasern besteht.

Der vorliegenden Erfindung liegt die Aufgabe zugrunde, ein Wattestäbchen der eingangs genannten Art dahingehend zu verbessern, dass es eine weichere Anfühlung vermittelt, jedoch ohne dass die Festigkeit des Wattekopfs wesentlich herabgesetzt wird. Ferner soll die Reinigungswirkung gegenüber solchen Wattestäbchen, die zu 100% aus Baumwolle bestehen, verbessert werden.

Diese Aufgabe wird bei einem gattungsgemäßen Wattestäbchen erfindungsgemäß dadurch gelöst, dass das Fasermaterial Mikrostapelfasern mit einer Länge von wenigstens 7 mm umfasst.

Wenn vorstehend von Mikrostapelfasern die Rede ist, so werden hierunter synthetische Fasern einer Faserstärke von < 1 dtex verstanden. Der Begriff der Mikrostapelfasern bringt dabei zum Ausdruck, dass für die Herstellung der Faservlieslage oder eines band- oder schnurförmigen Faservlieses zur Herstellung des Wattekopfs an den freien Enden der Wattestäbchen zuvor in einem separaten Herstellungsverfahren gebildete Mikrofasern einer bestimmten Länge oder eines bestimmten Längenbereichs verwendet werden. Diese vereinzelten Spatelfasern werden dann zur Bildung des Fasermaterials abgelegt. Es werden also nicht im Spinnvlies- oder Meltblown-Verfahren gebildete Fasern verwendet, bei denen (vgl. US 4,100,342) der Faserbildungsprozess und der Vliesbildungsprozess quasi in einem Vorgang stattfinden, sondern es werden in einem vom Vliesbildungsprozess separaten Herstellungsverfahren gebildete Mikrofasern einer bestimmten Länge oder eines bestimmten Längenbereichs (> 7 mm) verwendet. Es hat sich gezeigt, dass synthetische Mikrostapelfasern einem Wattestäbchen bzw. dem Wattekopf eines Wattestäbchens zu größerer Weichheit und damit zu einer hervorragenden Anfühlungswahrnehmung beim Benutzer zu verhelfen vermögen. Überraschenderweise ist damit aber nicht ein Rückgang der Reinigungs- oder Abschminkwirkung oder der Festigkeit des Wattekopfes verbunden. Im Gegenteil weisen erfindungsgemäße Wattestäbchen mit Mikrostapelfasern einer Länge von wenigstens 7 mm eine überlegene Reinigungs- und Abschminkwirkung auf, und sie sind besser geeignet, z. B. fetthaltigen Schmutz aus dem Ohr zu entfernen, als dies bei aus 100% Baumwollfasern bestehenden Wattestäbchen der Fall ist.

Aufgrund der Feinheit der Mikrostapelfasern ist die zur Verfügung stehende Oberfläche des Fasermaterials, welches in Kontakt mit der zu reinigenden Hautoberfläche treten kann, auch größer als bei reinen Baumwoll-Wattestäbchen. Diese große Oberfläche begrenzt demzufolge auch eine riesige Anzahl von Mikrospalten und -öffnungen, welche zur Aufnahme von Unreinheiten, Hautschuppen, fetthaltigem Schmutz oder Schminke dienen können.

Die Faserlänge der verwendbaren Mikrostapelfasern beträgt vorzugsweise 10 bis 38 mm, insbesondere 15 bis 32 mm.

Bei den Mikrostapelfasern kann es sich in weiterer Ausbildung der Erfindung um Polyester (PES) oder um Viskosefasern handeln.

Der Anteil der Mikrostapelfasern an der Masse des Fasermaterials beträgt insbesondere 3 bis 50 Gew.-%, insbesondere 5 bis 30 Gew.-% und vorzugsweise 5 bis 20 Gew.-%.

Es kann sich des Weiteren als vorteilhaft erweisen, wenn das Fasermaterial zusätzlich bis zu 97 Gew.-%, insbesondere 60 bis 97 Gew.-% und vorzugsweise 70 bis 95 Gew.-% Baumwollfasern umfasst. Die Einbeziehung von Baumwollfasern erweist sich insbesondere dann als vorteilhaft, wenn sich das Wattestäbchen beispielsweise zur Aufnahme einer gesichtsreinigenden Lösung eignen soll oder zum Abschminken unter Verwendung eines einen hohen Flüssigkeitsanteil aufweisenden Abschminkmittels eingesetzt werden soll. Als Baumwollfasern werden vorzugsweise Baumwollkämmlinge verwendet.

Zur Herstellung des erfindungsgemäßen Wattestäbchens werden die in einem separaten Prozess hergestellten synthetischen Mikrostapelfasern zur Vliesbildung nach an sich üblichen Vliesbildungsverfahren abgelegt. Werden verschiedene Faserarten verwandt, so werden diese zuvor vorzugsweise in einem Luftstrom vermischt und dann abgelegt. Sind zusätzlich wärmeschmelzbare Bindefasern vorhanden, so kann in einem "Air-through-Verfahren" mit genau einstellbarer Gastemperatur eine Thermofixierung des Vlieses vorgenommen werden, vorzugsweise ohne dass dabei die synthetischen Mikrostapelfasern thermisch und damit strukturell beeinflusst oder beeinträchtigt werden.

Im Hinblick auf die Erreichung einer hohen inneren Festigkeit des Fasermaterials im Wattekopf erweist es sich als vorteilhaft, wenn das Fasermaterial wärmeschmelzbare Bindefasern umfasst. Der Anteil der wärmeschmelzbaren Bindefasern an der Masse des Fasermaterials beträgt insbesondere 5 bis 20 Gew.-% und vorzugsweise 5 bis 15 Gew.-%.

In weiterer Ausbildung der Erfindung kann es sich bei den Bindefasern um Mehrkomponentenfasern, insbesondere um Bikomponentenfasern, handeln mit einer höher schmelzenden Trägerkomponente und einer niedriger schmelzenden Bindekomponente.

Die Mehrkomponentenfasern, insbesondere Bikomponentenfasern, haben in vorteilhafter Weise eine Faserstärke von 1,3 - 10 dtex, insbesondere von 1,3 - 3 dtex und eine Faserlänge von 3 bis 60 mm. Vorteilhafterweise kommen Kern/Mantel-Fasern oder Seite-an-Seite-Fasern zum Einsatz.

Es hat sich als vorteilhaft erwiesen, wenn Bikomponentenfasern mit einem Copolyester (CO-PES) als niedrig schmelzender Komponente und Polyester (PES) als höher schmelzender Komponente verwendet werden.

In weiterer Ausbildung der Erfindung von besonderer Bedeutung ist der Schmelzpunkt der wärmeschmelzbaren Bindefasern oder der niedrig schmelzenden Komponente (z.B. CO-PES) der Mehrkomponentenfasern geringer als der Schmelzpunkt der Mikrostapelfasern. Bspw. könnten Mikrostapelfasern aus einem Polyestermaterial verwendet werden mit einem Schmelzpunkt von etwa 256°C und CO-PES/PES-Bikomponentenfasern mit einem Schmelzpunkt der niedrig schmelzenden Komponente CO-PES von 110°C und der höher schmelzenden Komponente PES von 255°C. Solchenfalls könnte eine thermische Verfestigung des Faservlieses zur Herstellung des Wattepkopfs durchgeführt werden, ohne die höher schmelzende Komponente der Bikomponentenfasern und die Mikrostapelfasern thermisch zu verändern.

In weiterer Ausbildung der Erfindung von besonderer Bedeutung beträgt der Abzugswiderstand des den Wattekopf bildenden Fasermaterials, der ein Maß für die Anbindung des Fasermaterials an das freie Ende des Stiels aber auch für den Faserzusammenhalt darstellt, gemessen nach dem nachfolgend angegebenen Abzugstest wenigstens 30 N, vorzugsweise wenigstens 40 N.

Abzugstest:

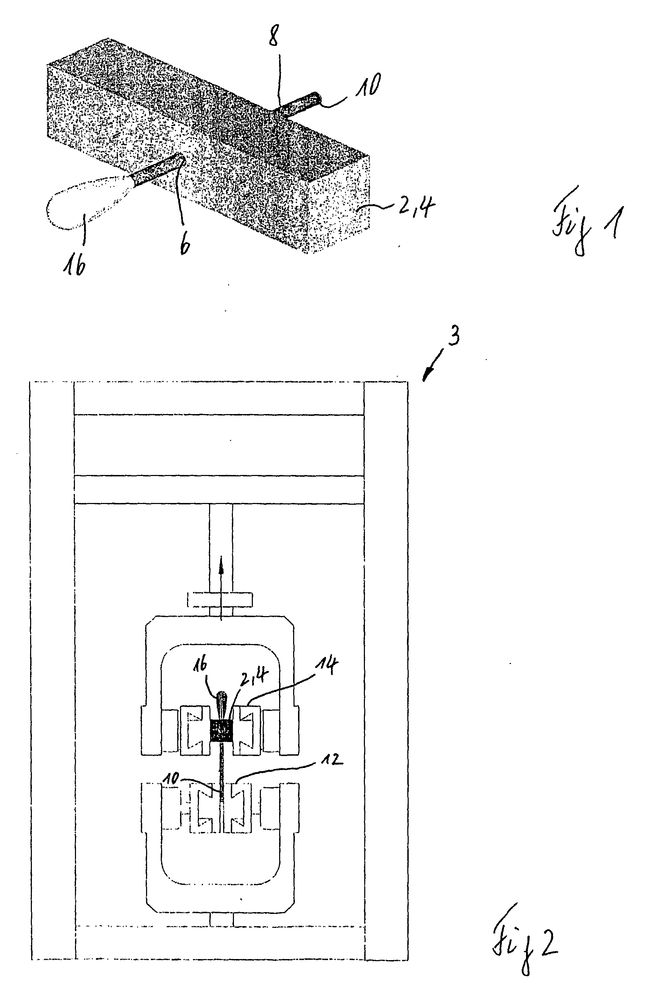

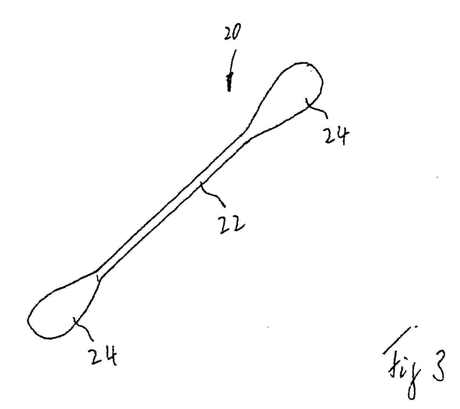

Mit Hilfe des Abzugstests wird der Abzugswiderstand, oder die Abzugsfestigkeit eines Wattekopfs beim Lösen vom freien Ende des Stiels eines kosmetischen oder medizinischen Wattestäbchens bestimmt. Hierfür werden ein aus Figur 1 ersichtlicher Probenhalter 2 und ein aus Figur 2 ersichtliches Zugprüfgerät 3 nach DIN 51221 verwendet. Der Probenhalter 2 umfasst einen Halteblock 4 mit einer Bohrung 6, deren Durchmesser geringfügig größer als der Außendurchmesser des Stiels 8 des Wattestäbchens ist. Der Durchmesser der Bohrung 6 ist dabei um 3 bis 5% des Außendurchmessers des Stiels 8 Wattestäbchens größer als dieser Außendurchmesser, so dass sich auch bei einer Toleranz des Außendurchmessers des Stiels von ± 3% der Stielbereich des Wattestäbchens immer noch im wesentlichen widerstandsfrei und gleitverschieblich in der Bohrung hin- und herbewegen lässt. Für die Durchführung des Tests wird von einem Wattestäbchen an der nicht zu prüfenden Seite der Wattekopf abgetrennt, und das Wattestäbchen wird mit diesem freien Ende durch die Bohrung 6 des Halteblocks 4 hindurch gesteckt. Der Probenhalter 2 mit dem zu prüfenden Stäbchen wird nun, wie aus Figur 2 ersichtlich, in dem Zugprüfgerät 3 eingespannt. Dabei wird das freie Ende 10 des Wattestäbchens, welches durch den Probenhalter 2 hindurch gesteckt ist, in der unteren Klemme 12 des Zugprüfgeräts 3, wie aus Figur 2 ersichtlich, fixiert. Der Probenhalter 2 selbst wird in der oberen Klemme 14 des Zugprüfgeräts fixiert. Das Zugprüfgerät ist so eingestellt, dass sich die Klemmen 12, 14 mit einer Prüfgeschwindigkeit von 300 mm/min auseinander bewegen, wobei die obere Klemme 14, wie dargestellt, von der ortsfesten unteren Klemme 12 mit der Prüfgeschwindigkeit entfernt wird. Es wird währenddessen die zwischen den Klemmen wirkende Prüfkraft gemessen. Es wird dann die während der Prüfung ermittelte Höchstzugkraft in Newton, auf eine Dezimale gerundet, angegeben. Eine jeweilige Prüfung endet damit, dass der Stiel 8 des Wattestäbchens von dem Wattekopf 16 abgeschert und durch die Bohrung 6 des Halteblocks 4 des Probenhalters 2 gezogen wird.

Im Hinblick auf eine optimale Saugfähigkeit erweist es sich als vorteilhaft, wenn das Fasermaterial des erfindungsgemäßen Wattestäbchens eine Absinkdauer in wässriger Lösung nach dem nachfolgend zu beschreibenden Absinkdauertest von wenigstens 3 sec., bevorzugt von wenigstens 3,4 sec., besonders bevorzugt von wenigstens 4 sec., ganz besonders bevorzugt von wenigstens 4,5 sec. aufweist.

Des Weiteren erweist es sich als vorteilhaft, wenn das Wasserhaltevermögen des Fasermaterials nach dem hier beschriebenen Saugfähigkeitstest wenigstens 21 g/g, insbesondere wenigstens 23 g/g beträgt.

Beide Tests werden an dem Fasermaterial im abgelegten Zustand durchgeführt, wie es dann unmittelbar zur Herstellung des Wattekopfs eingesetzt werden kann.

Je rascher das Fasermaterial in wässriger Flüssigkeit absinkt, desto hydrophiler ist das Fasermaterial insgesamt. Dies ist jedoch insbesondere bei der Reinigung fettiger Hautoberflächen nicht uneingeschränkt wünschenswert. Es hat sich gezeigt, dass bei Fasermaterialien, die Mikrofasern umfassen, mit einer Absinkdauer von mehr als 3 sec eine sehr viel bessere Reinigungswirkung erzielt werden kann, als bei reinen Baumwollfasermaterialien. Dies bedeutet aber nicht, dass mit einer etwas höheren Absinkdauer zwangsläufig ein schlechteres Wasserhaltevermögen verbunden ist. Mit erfindungsgemäßen Wattestäbchen lassen sich bei höherer Weichheit vielmehr höhere Saugfähigkeiten oder ein zumindest gleich hohes Wasserhaltevermögen erreichen als etwa mit reinen Baumwoll-Wattestäbchen, deren Wasserhaltevermögen in dem nachfolgenden zu beschreibenden Test im Bereich unterhalb von 22,5 g/g liegt.

Absinkdauertest und Saugfähigkeitstest:

Der folgende Test ist beschrieben in PH.EUR.1997, Monografie Verbandwatte aus Baumwolle; es handelt sich um einen Test der Saugfähigkeit unter Bestimmung der Absinkdauer eines mit Fasermaterial befüllten Drahtkörbchens in einer Flüssigkeit und des Wasserhaltevermögens. Das hierzu verwendbare Drahtkörbchen ist ein zylindrischer Korb aus Kupferdraht mit einem Drahtdurchmesser von 0,4 mm. Die Höhe beträgt 80 mm, der Durchmesser 50 mm, die Maschenweite 15 - 20 mm und die Masse 2,7 +/- 0,3 g. Ferner findet ein Becherglas mit Durchmesser 11 - 12 cm Verwendung.

Zu testendes Fasermaterial in abgelegter Vliesform wird in einer Prüfmenge von 5 g in das Drahtkörbchen eingelegt. Zuvor wurde der Korb auf 0,01 g genau gewogen (M1). Die 5 g Probenmaterial bilden die Masse M2. Das Becherglas wird mit demineralisiertem Wasser bis zu einer Höhe von etwa 100 mm gefüllt und der gefüllte Korb wird aus einer Höhe von 10 mm auf das Wasser fallen gelassen. Mit einer Stopuhr wird die Zeit bis zum Absinken unter die Wasseroberfläche bestimmt, also die Absinkdauer. Unmittelbar nach der Bestimmung der Absinkdauer wird der Korb aus dem Wasser gehoben und zum Abtropfen 30 s lang in seiner Längsachse horizontal gehalten. Nach Ablauf der Abtropfzeit wird der Korb in ein tariertes Becherglas (M3) gegeben und auf 0,01 g genau gewogen (M4).

Das Wasserhaltevermögen ergibt sich

$$\text{in } g/g = \frac{M4 - (M2 + M3)}{M2 - M1}$$

Die Absinkdauer und das Wasserhaltevermögen werden als Mittelwert aus zumindest drei Bestimmungen angegeben. Für bevorzugtes Fasermaterial für die Herstellung von Wattestäbchen beträgt die Absinkdauer wenigstens 3 sec und das Wasserhaltevermögen beträgt wenigstens 21 g/g. Dies lässt sich durch den Anteil absorbierender Fasern und/oder durch Zusatz von Hydrophilierungsmittel erreichen.

In weiterer Ausbildung der Erfindung erweist es sich als vorteilhaft, wenn das Fasermaterial Weichmacher enthält, und zwar vorzugsweise zu wenigstens 0,2 Gew.-% der Masse des Fasermaterials. Die Weichmacher finden vorzugsweise zur Behandlung von zugesetzten Baumwollfasern Verwendung. Es können Weichmacher verwendet werden, die ein Fettsäurekondensationsprodukt und/oder funktionelle Polydimethylsiloxane und/oder Polyäthylene umfassen.

Besonders gute Qualitäten des erfindungsgemäßen Wattestäbchens werden erreicht, wenn dessen den Wattekopf bildendes Fasermaterial hergestellt ist aus einem Faservliesstreifen oder einer Fasrvliesschnur eines Gewichts von 0,5 bis 8 g/m, insbesondere 1 bis 2 g/m. Dabei weist das Faservlies insbesondere eine Breite von 10-20 mm, vorzugsweise von 12-18 mm auf. Der im Herstellungsprozess zugeführte schnur- oder streifenförmige Abschnitt des Faservlieses ist damit sehr dünn und vorzugsweise auch sehr schmal und lässt sich hervorragend um das freie Ende des Stiels herumlegen und in an sich bekannter Weise dort befestigen.

Es erweist sich als ganz besonders vorteilhaft, wenn das Faservlies oder der Faservliesstreifen zur Herstellung des Wattekopfs ein Kardenvlies mit in Maschinenrichtung verlaufender Orientierung ist. Auf diese Weise kann eine hohe Zugfestigkeit in dem schnur- oder streifenförmigen Faservlies erreicht werden, was die Herstellung des Wattekopfs am freien Ende des Stiels erleichtert.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung. In der Zeichnung zeigt:

Figur 1   eine perspektivische Ansicht eines Probenhalters für ein Wattestäbchen zur Durchführung des Abzugstests;

Figur 2   eine schematische Ansicht des Zugprüfgeräts zur Durchführung des Abzugstests und

Figur 3   eine Ansicht eines kosmetischen Wattestäbchens.

Die Figuren 1 und 2 wurden bereits vorausgehend erläutert.

Figur 3 zeigt ein typisches kosmetisches Wattestäbchen 20 mit einem Stiel 22 und einem Wattekopf 24 an beiden Enden des Stiels 22 aus einem Fasermaterial mit Mikrostapelfasern einer Länge von wenigstens 7 mm und einer Faserstärke von ≤ 1 dtex.

Nach einer bevorzugten Zusammensetzung des Fasermaterials umfasst dieses zu 3 bis 50 Gew.-%, insbesondere zu 5 bis 30 Gew.-% und vorzugsweise 5 bis 20 Gew.-% Mikrostapelfasern einer Stärke von 0,9 dtex. Ferner umfasst das Fasermaterial bis zu 97 Gew.-%, insbesondere 60 bis 95 und vorzugsweise 70 bis 95 Gew.-% Baumwolle. Bei den vorstehend erwähnten Mikrofasern handelt es sich um Polyester oder Viskosefasern mit einer Faserlänge von 18 bis 38 mm.

Nach einer besonders bevorzugten Ausführungsform umfasst das Fasermaterial 90 Gew.-% Baumwollfasern, vorzugsweise Baumwollkämmlinge, und 10 Gew.-% Mikrostapelfasern. Zur Bildung eines Wattekopfs werden etwa 0,02 bis 0,3 g, insbesondere 0,02 bis 0,05 g Fasermaterial verwendet. Der Kopfdurchmesser, gemessen quer zur Längsrichtung des Stiels, beträgt 3 bis 12 mm, insbesondere 3,5 bis 5,5mm.

Die Köpfchenlänge, gemessen in Längsrichtung des Stiels beträgt 10 bis 35 mm, insbesondere 11 bis 16 mm. Der Stieldurchmesser beträgt etwa 2,4 bis 5,5 mm.

Die nachfolgende Tabelle zeigt Messwerte des Absinkdauertests und des Saugfähigkeitstests (Wasserhaltevermögen), wobei vier Proben einer Mischung aus 10 Gew.-% Polyestermikrofasern einer Stärke von 0,9 dtex und 90 Gew.-% Baumwollfasern sowie drei Proben einer Mischung aus 25 Gew.-% Polyestermikrofasern einer Stärke von 0,9 dtex und 75 Gew.-% Baumwollfasern untersucht und mit einer Probe einer 100% Baumwolle-Watteschnur verglichen wurden. Bereits der Zusatz von 10% Mikrostapelfasern bewirkt bei höherer Weichheit eine Zunahme der Absinkdauer und eine Erhöhung des Wasserhaltevermögens.

| Prüfmerkmale | Einheit | Watteschnur aus 100 % BW | Watteschnur aus 90 % BW / 10 % Mikrofaser | | | | Watteschnur aus 75 % BW / 25 % Mikrofaser | | |
|---|---|---|---|---|---|---|---|---|---|
| | | | Probe 1 | Probe 2 | Probe 3 | Probe 4 | Probe 1 | Probe 2 | Probe 3 |
| Absinkdauer | sec | 3,02 | 3,75 | 4,33 | 3,58 | 3,70 | 5,33 | 4,78 | 4,52 |
| | | 2,95 | 3,70 | 3,56 | 3,04 | 3,79 | 4,97 | 4,24 | 4,71 |
| | | 2,95 | 3,31 | 3,65 | 3,96 | 3,71 | 5,14 | 5,47 | 4,34 |
| | | 3,16 | 3,52 | 3,72 | 3,36 | 3,64 | 4,92 | 4,95 | 5,05 |
| | | 2,90 | 3,64 | 3,49 | 3,14 | 2,99 | 4,84 | 5,08 | 5,34 |
| x / s | | 3,0/0,1 | 3,6/0,2 | 3,8/0,3 | 3,4/0,4 | 3,6/0,3 | 5,0/0,2 | 4,9/0,4 | 4,8/0,4 |
| Wasserhalte-vermögen | g/g | 21,9 | 24,4 | 22,7 | 22,3 | 22,4 | 25,1 | 25,6 | 24,2 |
| | | 22,4 | 24,7 | 23,6 | 19,6 | 21,7 | 26,1 | 23,1 | 25,8 |
| | | 21,3 | 23,1 | 22,3 | 22,6 | 23,0 | 25,1 | 26,0 | 23,8 |
| | | 22,7 | 23,4 | 22,8 | 22,0 | 23,4 | 24,2 | 24,8 | 25,3 |
| | | 22,0 | 25,2 | 23,1 | 21,3 | 21,6 | 26,4 | 26,0 | 26,3 |
| x / s | | 22/0,5 | 24,2/0,9 | 22,9/0,5 | 21,6/1,2 | 22,4/0,8 | 25/0,9 | 25/1,2 | 25/1,1 |

BW: Baumwollkämmlinge
X: arithemtisches Mittel
S: Standardabweichung

EP 1 476 597 B1

**Patentansprüche**

1. Wattestäbchen für kosmetische oder medizinische Zwecke oder für die Körperpflege, mit einem Stiel, welcher einen Wattekopf bildendes Fasermaterial an zumindest einem seiner Enden aufweist, **dadurch gekennzeichnet, dass** das Fasermaterial Mikrostapelfasern einer Länge von wenigstens 7 mm umfasst.

2. Wattestäbchen nach Anspruch 1, **dadurch gekennzeichnet, dass** es sich bei den Mikrostapelfasern um Polyester (PES)- oder um Viskosefasern handelt.

3. Wattestäbchen nach Anspruch 1 oder 2, **dadurch gekennzeichnet, dass** der Anteil der Mikrostapelfasern an der Masse des Fasermaterials 3 - 50 Gew.-%, insbesondere 5 - 30 Gew.-% und vorzugsweise 5 - 20 Gew.-% beträgt.

4. Wattestäbchen nach einem der vorstehenden Ansprüche, **dadurch gekennzeichnet, dass** das Fasermaterial zusätzlich bis zu 97 Gew.-%, insbesondere 60 - 97 Gew.-% und vorzugsweise 70 - 95 Gew.-% Baumwollfasern umfasst.

5. Wattestäbchen nach Anspruch 4, **dadurch gekennzeichnet, dass** die Baumwollfasern Baumwollkämmlinge sind.

6. Wattestäbchen nach einem der vorstehenden Ansprüche, **dadurch gekennzeichnet, dass** das Fasermaterial wärmeschmelzbare Bindefasern umfasst.

7. Wattestäbchen nach Anspruch 6, **dadurch gekennzeichnet, dass** der Anteil der wärmeschmelzbare Bindefasern an der Masse des Fasermaterials 5 - 20 Gew.-%, insbesondere 5 - 15 Gew.-% beträgt.

8. Wattestäbchen nach Anspruch 6 oder 7, **dadurch gekennzeichnet, dass** die wärmeschmelzbaren Bindefasern Mehrkomponentenfasern, insbesondere Bikomponentenfasern sind.

9. Wattestäbchen nach Anspruch 8, **dadurch gekennzeichnet, dass** die Mehrkomponentenfasern eine Faserstärke von 1,3 - 10 dtex, insbesondere 1,3 - 3 dtex aufweisen.

10. Wattestäbchen nach Anspruch 8 oder 9, **dadurch gekennzeichnet, dass** die Mehrkomponentenfasern eine Faserlänge von 3 - 60 mm aufweisen.

11. Wattestäbchen nach einem der Ansprüche 6 - 10, **dadurch gekennzeichnet, dass** die Bindefasern Copolyester (CO-PES) / Polyester (PES)- Bikomponentenfasern sind.

12. Wattestäbchen nach einem der Ansprüche 8 - 11, **dadurch gekennzeichnet, dass** der Schmelzpunkt der wärmeschmelzbaren Bindefasern oder der niedrig schmelzenden Komponente der Mehrkomponentenfasern geringer ist als der Schmelzpunkt der Mikrostapelfasern.

13. Wattestäbchen nach einem der vorstehenden Ansprüche, **dadurch gekennzeichnet, dass** der Abzugswiderstand des Fasermaterials vom freien Ende des Stiels gemessen nach dem hier angegebenen Abzugstest größer als 30 N, vorzugsweise größer als 40 N ist.

14. Wattestäbchen nach einem der vorstehenden Ansprüche, **dadurch gekennzeichnet, dass** die Absinkdauer des Fasermaterials in wässriger Lösung nach dem hier beschriebenen Absinkdauertest wenigstens 3 sec, vorzugsweise wenigstens 3,4 sec, besonders bevorzugt wenigstens 4 sec und ganz besonders bevorzugt wenigstens 4,5 beträgt.

15. Wattestäbchen nach einem der vorstehenden Ansprüche, **dadurch gekennzeichnet, dass** das Wasserhaltevermögen des Fasermaterials nach dem hier beschriebenen Saugfähigkeitstest wenigstens 21 g/g, insbesondere wenigstens 23 g/g beträgt (g Flüssigkeit je g Fasermaterial).

16. Wattestäbchen nach einem der vorstehenden Ansprüche, **dadurch gekennzeichnet, dass** das Fasermaterial einen Weichmacher enthält.

17. Wattestäbchen nach einem der vorstehenden Ansprüche, dessen den Wattekopf bildendes Fasermaterial hergestellt ist aus einem Faservlies oder einem Faservliesstreifen eines Gewichts von 0,5 - 8 g/m, insbesondere 1 - 2 g/m.

18. Wattestäbchen nach Anspruch 17, **dadurch gekennzeichnet, dass** das Faservlies oder der Faservliesstreifen ein Kardenvlies mit in Maschinenrichtung verlaufender Orientierung ist.

**Claims**

1. Cotton bud for cosmetic or medical purposes or for personal hygiene, comprising a stem, which comprises at at least one of its ends a fibrous material forming a cotton head, **characterised in that** the fibrous material comprises micro-staple fibres having a length of at least 7 mm.

2. Cotton bud according to claim 1, **characterised in that** the micro-staple fibres are polyester (PES) or viscose fibres.

3. Cotton bud according to either claim 1 or claim 2, **characterised in that** the micro-staple fibre content of the mass of fibrous material is 3 to 50 % by weight, in particular 5 to 30 % by weight and preferably 5 to 20 % by weight.

4. Cotton bud according to any one of the preceding claims, **characterised in that** the fibrous material also comprises up to 97 % by weight, in particular 60 to 97 % by weight and preferably 70 to 95 % by weight of cotton fibres.

5. Cotton bud according to claim 4, **characterised in that** the cotton fibres are cotton noils.

6. Cotton bud according to any one of the preceding claims, **characterised in that** the fibrous material comprises heat-meltable binding fibres.

7. Cotton bud according to claim 6, **characterised in that** the heat-meltable binding fibre content of the mass of fibrous material is 5 to 20 % by weight, in particular 5 to 15 % by weight.

8. Cotton bud according to either claim 6 or claim 7, **characterised in that** the heat-meltable binding fibres are multi-component fibres, in particular bi-component fibres.

9. Cotton bud according to claim 8, **characterised in that** the multi-component fibres have a fibre thickness from 1.3 to 10 dtex, in particular from 1.3 to 3 dtex.

10. Cotton bud according to either claim 8 or claim 9, **characterised in that** the multi-component fibres have a fibre length from 3 to 60 mm.

11. Cotton bud according to any one of claims 6 to 10, **characterised in that** the binding fibres are copolyester (CO PES)/polyester (PES) bi-component fibres.

12. Cotton bud according to any one of claims 8 to 11, **characterised in that** the melting point of the heat-meltable binding fibres or of the low-melting components of the multi-component fibres is lower than the melting point of the micro-staple fibres.

13. Cotton bud according to any one of the preceding claims, **characterised in that** the take-off resistance of the fibrous material from the free end of the stem, measured according to the take-off test indicated in the present document, is greater than 30 N, preferably greater than 40 N.

14. Cotton bud according to any one of the preceding claims, **characterised in that** the sinking time of the fibrous material in aqueous solution, according to the sinking time test described in the present document, is at least 3 sec, preferably at least 3.4 sec, particularly preferably at least 4 sec and very particularly preferably at least 4.5 sec.

15. Cotton bud according to any one of the preceding claims, **characterised in that** the water retention value of the fibrous material according to the absorption capacity test described in the present document, is at least 21 g/g, in particular at least 23 g/g (g liquid per g fibrous material).

16. Cotton bud according to any one of the preceding claims, **characterised in that** the fibrous material comprises a softener.

17. Cotton bud according to any one of the preceding claims, of which the fibrous material forming the cotton head is produced from a nonwoven or a nonwoven strip having a mass 0.5 to 8 g/m, in particular 1 to 2 g/m.

18. Cotton bud according to claim 17, **characterised in that** the nonwoven or the nonwoven strip is a card web orientated in the direction of the machine.

## Revendications

1. Coton-tige pour applications cosmétiques ou médicales ou pour l'hygiène corporelle, comprenant une tige qui présente une matière fibreuse formant une tête en ouate à au moins une de ses extrémités, **caractérisé en ce que** la matière fibreuse comprend des microfibres discontinues d'une longueur d'au moins 7 mm.

2. Coton-tige selon la revendication 1, **caractérisé en ce que** les microfibres discontinues sont des fibres de polyester (PES) ou de viscose.

3. Coton-tige selon la revendication 1 ou 2, **caractérisé en ce que** la part des microfibres discontinues dans la masse de la matière fibreuse est comprise entre 3 et 50 % en poids, en particulier entre 5 et 30 % en poids et de préférence entre 5 et 20 % en poids.

4. Coton-tige selon l'une des revendications précédentes, **caractérisé en ce que** la matière fibreuse comprend en supplément des fibres de coton représentant jusqu'à 97 % en poids, en particulier de 60 à 97 % en poids et de préférence de 70 à 95 % en poids.

5. Coton-tige selon la revendication 4, **caractérisé en ce que** les fibres de coton sont des débourrures de coton.

6. Coton-tige selon l'une des revendications précédentes, **caractérisé en ce que** la matière fibreuse comporte des fibres de liaison thermofusibles.

7. Coton-tige selon la revendication 6, **caractérisé en ce que** la part des fibres de liaison thermofusibles dans la masse de la matière fibreuse est comprise entre 5 et 20 % en poids, en particulier entre 5 et 15% en poids.

8. Coton-tige selon la revendication 6 ou 7, **caractérisé en ce que** les fibres de liaison thermofusibles sont des fibres à plusieurs composants, en particulier des fibres à deux composants.

9. Coton-tige selon la revendication 8, **caractérisé en ce que** les fibres à plusieurs composants présentent une épaisseur de fibres comprise entre 1,3 et 10 dtex, en particulier entre 1,3 et 3 dtex.

10. Coton-tige selon la revendication 8 ou 9, **caractérisé en ce que** les fibres à plusieurs composants présentent une longueur de fibres comprise entre 3 et 60 mm.

11. Coton-tige selon l'une des revendications 6 à 10, **caractérisé en ce que** les fibres de liaison sont des fibres à deux composants copolyester (CO-PES) / polyester (PES).

12. Coton-tige selon l'une des revendications 8 à 11, **caractérisé en ce que** le point de fusion des fibres de liaison thermofusibles ou du composant des fibres à plusieurs composants ayant le point de fusion le plus bas est inférieur au point de fusion des microfibres discontinues.

13. Coton-tige selon l'une des revendications précédentes, **caractérisé en ce que** la résistance à l'enlèvement de la matière fibreuse à partir de l'extrémité libre de la tige, mesurée suivant l'essai d'enlèvement indiqué ici est supérieure à 30 N, de préférence supérieure à 40 N.

14. Coton-tige selon l'une des revendications précédentes, **caractérisé en ce que** la durée d'enfoncement de la matière fibreuse dans une solution aqueuse suivant l'essai de durée d'enfoncement décrit ici est d'au moins 3 secondes, de préférence d'au moins 3,4 secondes, en particulier de préférence d'au moins 4 secondes et de façon particulièrement préférée d'au moins 4,5 secondes.

15. Coton-tige selon l'une des revendications précédentes, **caractérisé en ce que** la capacité de rétention d'eau de la matière fibreuse suivant l'essai de pouvoir absorbant décrit ici est d'au moins 21 g/g, en particulier d'au moins 23 g/g (g de liquide par g de matière fibreuse).

**16.** Coton-tige selon l'une des revendications précédentes, **caractérisé en ce que** la matière fibreuse contient un assouplissant.

**17.** Coton-tige selon l'une des revendications précédentes, **caractérisé en ce que** sa matière fibreuse formant la tête en ouate est fabriquée à partir d'un non-tissé ou d'une bande de non-tissé d'un poids compris entre 0,5 et 8 g/m, en particulier entre 1 et 2 g/m.

**18.** Coton-tige selon la revendication 17, **caractérisé en ce que** le non-tissé ou la bande de non-tissé est un non-tissé de carde avec une orientation s'étendant dans la direction de la machine.

Fig 1

Fig 2

20

24

22

24

fig 3